# EUROPEAN PATENT SPECIFICATION

(11) **EP 1 703 877 B1**
(45) Date of publication and mention of the grant of the patent: **20.08.2008**
(21) Application number: 05703046.2
(22) Date of filing: 03.01.2005
(51) Int. Cl.: A61F 9/00

(54) **EYE WALL ANCHORED FIXTURES**
VERANKERTE AUGENWAND-VORRICHTUNGEN
DISPOSITIFS FIXES ANCRES DANS LA PAROI OCULAIRE

(30) Priority: 12.01.2004 IL 15981804
(43) Date of publication of application: 27.09.2006
(73) Proprietor: Nulens Ltd, Herzliya Pituach 46121 (IL)
(72) Inventor: BEN NUN, Joshua, 40291 D. N. VITKIN (IL)
(74) Representative: South, Nicholas Geoffrey
(86) International application number: PCT/IL2005/000003
(87) International publication number: WO 2005/065600

(56) References cited:
- WO-A-02/100318
- WO-A-03/092564
- SU-A1- 980 711
- US-A1- 2001 008 969
- US-A1- 2003 175 324
- US-B1- 6 299 895

## Description

### Field of the Invention

The invention pertains to eye wall anchored fixtures.

### Background of the Invention

Eye wall anchored fixtures are known in the art.

US Patent 5,098,443 to Parel et al. illustrates and describes intraocular and intraorbital implantable devices for the controlled release of pharmacological agents.

US Patent 5,466,233 to Weiner et al. illustrates and describes an intraocular drug delivery tack for transversing an eye's pars plana for administering a drug to the eye's vitreous cavity.

US Patent 5,830,173 to Avery et al illustrates and describes an intravitreal medicine delivery system device for administering a drug to an eye's vitreous cavity.

US Patent Application Publication No. US 2002/0110591 to Brubaker et al. illustrates and describes a sustained release drug delivery device for suturing to an eye wall.

WO 03/092564 discloses an eye wall anchored fixture comprising an anchor member for resting on or being countersunk into an eye wall and a support member mounted on said anchor member and traversing through the eye wall for supporting an intraocular device (4) in the eye's vitreous cavity.

### Summary of the Invention

The present invention is for eye wall anchored fixtures having at least one elongated anchor member for driven lengthwise insertion into an eye wall for supporting an intraocular device in the eye's vitreous cavity according to claim 1. The fixtures are intended for implantation in a circumferential band of an eye wall devoid of viable tissues called the pars plana. The width of an average adult eye's pars plana increases from a minimum width W_{N} of about 3.5mm towards a patient's nose to a maximum width W_{T} of about 4.5mm towards a patient's temporal region. The fixtures are preferably anchored temporally since an eye's pars plana is not only wider but also more accessible. The fixtures are intended to be anchored in an eye's pars plana in a transverse direction to its thickness. The fixtures can be designed for withdrawal, or as a self-anchoring implant, or as a sealing self-anchoring implant for sealing a throughgoing incision. Fixtures can be integrally formed with intraocular devices designed for intraocular drug administration, for acquiring intraocular physiological measurements for monitoring and/or diagnostic purposes, for example, a pressure sensor for monitoring glaucoma, and the like. Alternatively, fixtures can be formed with attachment devices whereby three or more fixtures deployed around an eye's pars plana are capable of supporting a relatively massive intraocular device within an eye's vitreous cavity.

### Brief Description of the Drawings

In order to understand the invention and to see how it can be carried out in practice, preferred embodiments will now be described, by way of non-limiting examples only, with reference to the accompanying drawings in which similar parts are likewise numbered, and in which:
Fig. 1 is a pictorial view showing an eye wall anchored L-shaped fixture with a drug release capsule for intraocular drug administration;
Fig. 2 is a pictorial view showing an eye wall anchored L-shaped fixture with an electronic sensor for acquiring intraocular physiological measurements;
Fig. 3 is a pictorial view showing an eye wall anchored L-shaped fixture for supporting a discrete intraocular device in an eye's vitreous cavity;
Fig. 4 is a pictorial view showing a self-anchoring member of a self-anchoring L-shaped fixture;
Fig. 5 is a pictorial view showing the oppositely directed self-anchoring members of a sealing self-anchoring T-shaped fixture;
Fig. 6 is a pictorial cross section showing Figure 1's fixture anchored in an eye's pars plana together with a close-up of the anchoring site;
Fig. 7 is a pictorial close-up showing the anchoring of a Figure 4 type fixture in an eye's pars plana;
Fig. 8 is a pictorial close-up showing the anchoring of a Figure 5 type fixture in an eye's pars plana; and
Fig. 9 is a pictorial view showing four Figure 5 type fixtures supporting an intraocular device in an eye's vitreous cavity.

### Detailed Description of Preferred Embodiments of the Present Invention

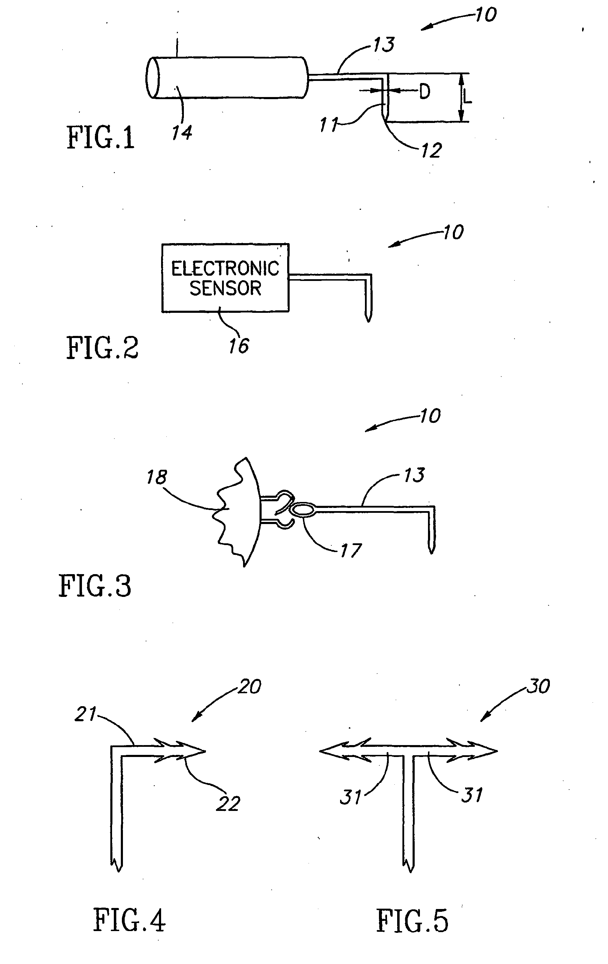

Figures 1-3 show eye wall anchored L-shaped fixtures 10 having a generally L-shaped structure including an elongated anchor member 11 with a pointed leading end 12, and a support member 13. The anchor members 11 have a length L in the range of about 1mm to about 2mm, and preferably 1.50mm±0.10mm, and a diameter D in the range of about 100µm to about 200µm, and preferably 150µm±10µm. The fixtures 10 are made from a biocompatible material suitable for implantation in an eye wall, and preferably stainless steel so as to be enable slight resilient deformation from the preferred right angle α=90° for facilitating some flexibility to deploy an intraocular device in a preferred location in an eye's vitreous cavity. The support member 13 can be provided with a drug release capsule 14 for intraocular drug administration (see Figure 1), an electronic sensor 16 for acquiring intraocular measurements for monitoring and/or diagnostic purposes (see Figure 2), and the like. Alternatively, a support member 13 can terminate in an attachment device 17 for supporting a discrete intraocular device 18 (see Figure 3). The attachment devices 17 can be implemented by loops, hooks, clips, and the like.

Figure 4 shows an eye wall anchored L-shaped fixture 20 having a similar construction as a fixture 10 but with a self-anchoring anchor member 21 having a self-anchoring leading end 22 for anchoring the fixture 20 in an eye's pars plana on its driven lengthwise insertion thereinto. The leading end 22 can be barbed, hook shaped, and the like.

Figure 5 shows an eye wall anchored T-shaped fixture 30 having a similar construction as a fixture 10 but with a pair of oppositely directed self-anchoring elongated anchor members 31. The fixture 30 can be employed for sealing a throughgoing incision through which it is implanted in an eye by virtue of the anchor members 31 being in serted into opposite side walls of the throughgoing incision so as to draw them together.

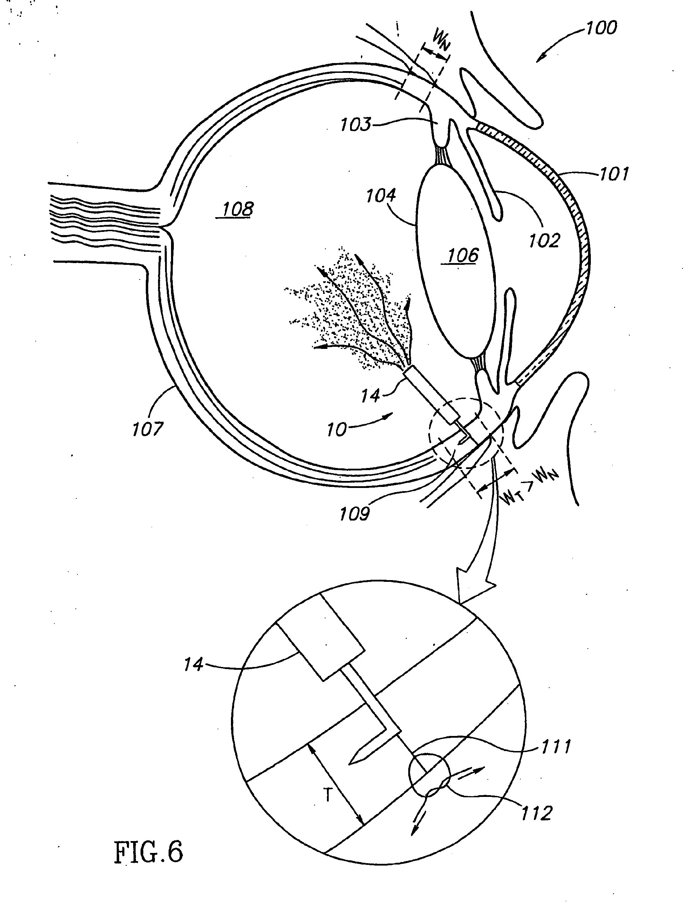

Figure 6 shows an eye 100 having a cornea 101, an iris 102, a ciliary body 103 supporting a capsular bag 104 with a natural lens 106, an eye wall 107, and a vitreous cavity 108. The eye wall 107 includes a circumferential pars plana 109 whose width increases from a minimum width W_{N} of about 3.5mm to a maximum width W_{T} of about 4.5mm, and which has a largely uniform thickness T of about 1mm. An incision 111 is made in the pars plana 109 and the fixture 10 is implanted in the eye 100 with its anchor member 11 driven lengthwise thereinto for supporting the drug release capsule 14 in the eye's vitreous cavity 108. The incision 111 is closed with a suture 112.

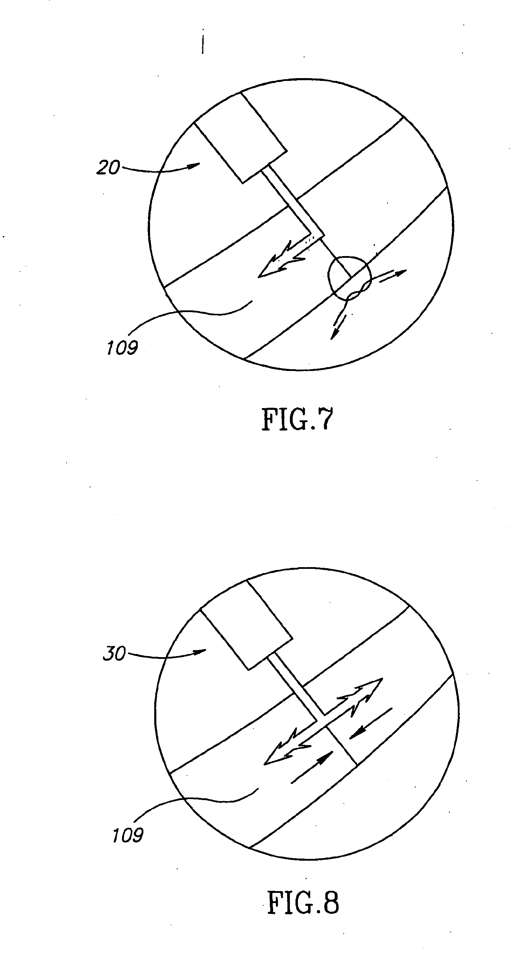

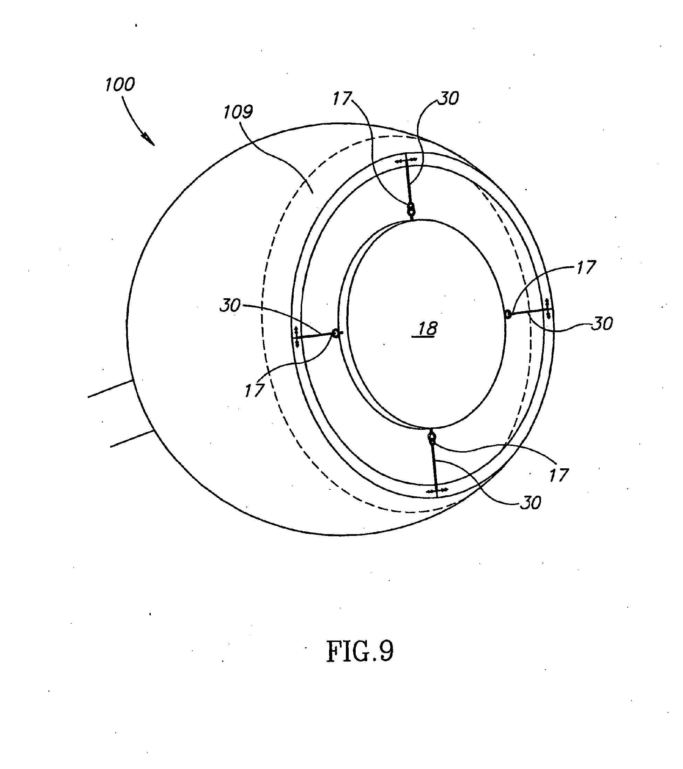

Figure 7 shows a fixture 20 anchored in an eye's pars plana 109 also with a suture for closing the incision. Figure 8 shows a fixture 30 anchored in an eye's pars plana 109 for drawing the side walls on either side of the incision towards one another for enabling the eye to seal itself without the need for a suture. Figure 9 shows four fixtures 30 with attachment devices 17 deployed at 0°, 90°, 180°, and 270° around an eye's pars plana 109 for supporting an intraocular device 18 in an eye's vitreous cavity.

While the invention has been described with respect to a limited number of embodiments, it will be appreciated that many variations, modifications, and other applications of the invention can be made within the scope of the appended claims.

## Claims

1. An eye wall anchored fixture (10,20,30) for implantation in an eye having an eye wall and a vitreous cavity, the fixture comprising an L-shaped element including at least one elongated anchor member (11) for driven lengthwise insertion into the eye wall in a transverse direction to the eye wall's thickness, each said at least one elongated anchor member (11) having a length in the range of about 1mm to about 2mm, and a diameter in the range of about 100µm to about 200µm, and a support member (13) mounted on said at least one elongated anchor member (11) for supporting an intraocular device m the eye's vitreous cavity.

2. The fixture (10,20,30) according to claim 1 wherein said length is in the range of 1.50mm±0.10mm.

3. The fixture (10,20,30) according to claim 1 or 2 wherein said diameter is in the range of 150µm±10µm.

4. The fixture (20) according to any one of claims 1 to 3 wherein said at least one elongated anchor member includes a single elongated anchor member (21) with a self-anchoring leading end (22) for anchoring the fixture (10) in an eye wall on its driven lengthwise insertion thereinto.

5. The fixture (30) according to any one of claims 1 to 3 wherein said at least one elongated anchor member includes a pair of oppositely directed elongated anchor members (31) each with a self-anchoring leading end whereby the fixture is capable of sealing a throughgoing incision in an eye wall via which it is implanted therein.

6. The fixture (10,20,30) according to any one of claims 1 to 5 wherein said support member (13) is integrally formed with said intraocular device.

7. The fixture (10,20,30) according to claim 6 wherein said intraocular device administers intraocular drugs.

8. The fixture (10,20,30) according to claim 6 wherein said intraocular device acquires intraocular measurements.

9. The fixture (10,20,30) according to any one of claims 1 to 5 wherein said support member includes an attachment device for supporting a discrete intraocular device in an eye's vitreous cavity.

## Patentansprüche

1. Verankerte Augenwand-Vorrichtung (10, 20, 30) zur Implantation in ein Auge mit einer Augenwand und einem Glaskörper, wobei die Vorrichtung ein L-förmiges Element umfasst, das mindestens ein längliches Verankerungsteil (11) zur getriebenen längsgerichteten Einführung in die Augenwand in einer Querrichtung zur Dicke der Augenwand, wobei jedes des mindestens einen länglichen Verankerungsteils (11) eine Länge im Bereich von etwa 1 mm bis etwa 2 mm und einen Durchmesser im Bereich von etwa 100 µm bis etwa 200 µm aufweist, und ein Halteteil (13), das an dem mindestens einen länglichen Verankerungsteil (11) montiert ist, zum Halten einer intraokularen Vorrichtung im Glaskörper des Auges beinhaltet.

2. Vorrichtung (10, 20, 30) nach Anspruch 1, wobei die Länge im Bereich von 1,50 mm ± 0,10 mm liegt.

3. Vorrichtung (10, 20, 30) nach Anspruch 1 oder 2, wobei der Durchmesser im Bereich von 150 µm ± 10 µm liegt.

4. Vorrichtung (20) nach einem der Ansprüche 1 bis 3, wobei das mindestens eine längliche Verankerungsteil ein einziges längliches Verankerungsteil (21) mit einem selbstverankernden Vorderende (22) zum Verankern der Vorrichtung (10) in einer Augenwand bei deren getriebenen längsgerichteten Einführung dorthinein beinhaltet.

5. Vorrichtung (30) nach einem der Ansprüche 1 bis 3, wobei das mindestens eine längliche Verankerungsteil ein Paar entgegengesetzt gerichteter länglicher Verankerungsteile (31), jeweils mit einem selbstverankernden Vorderende, beinhaltet, wodurch die Vorrichtung einen durchgehenden Schnitt in einer Augenwand, mittels dessen sie darin implantiert ist, verschließen kann.

6. Vorrichtung (10, 20, 30) nach einem der Ansprüche 1 bis 5, wobei das Halteteil (13) integral mit der intraokularen Vorrichtung ausgebildet ist.

7. Vorrichtung (10, 20, 30) nach Anspruch 6, wobei die intraokulare Vorrichtung intraokulare Arzneimittel abgibt.

8. Vorrichtung (10, 20, 30) nach Anspruch 6, wobei die intraokulare Vorrichtung intraokulare Messungen erfasst.

9. Vorrichtung (10, 20, 30) nach einem der Ansprüche 1 bis 5, wobei das Halteteil eine Befestigungsvorrichtung zum Halten einer separaten intraokularen Vorrichtung im Glaskörper eines Auges beinhaltet.

## Revendications

1. Dispositif de fixation (10, 20, 30) ancré dans la paroi oculaire pour l'implantation dans un oeil ayant une paroi oculaire et une humeur vitrée, le dispositif de fixation comprenant un élément en L comprenant au moins un élément d'ancrage allongé (11) pour l'insertion longitudinale entraînée dans la paroi oculaire dans une direction transversale à l'épaisseur de la paroi oculaire, chaque dit au moins un élément d'ancrage allongé (11) ayant une longueur dans la plage d'environ 1 mm à environ 2 mm, et un diamètre dans la plage d'environ 100 µm à environ 200 µm, et un élément de support (13) monté sur ledit au moins un élément d'ancrage allongé (11) pour supporter un dispositif intraoculaire dans l'humeur vitrée de l'oeil.

2. Dispositif de fixation (10, 20, 30) selon la revendication 1, dans lequel ladite longueur est dans la plage de 1,50 mm ± 0,10 mm.

3. Dispositif de fixation (10, 20, 30) selon la revendication 1 ou 2, dans lequel ledit diamètre est dans la plage de 150 µm ± 10 µm.

4. Dispositif de fixation (20) selon l'une quelconque des revendications 1 à 3, dans lequel ledit au moins un élément d'ancrage allongé comprend un élément d'ancrage allongé unique (21) avec une extrémité avant à ancrage automatique (22) pour ancrer le dispositif de fixation (10) dans une paroi oculaire lors de son insertion longitudinale entraînée dans celle-ci.

5. Dispositif de fixation (30) selon l'une quelconque des revendications 1 à 3, dans lequel ledit au moins un élément d'ancrage allongé comprend une paire d'éléments d'ancrage allongés dirigés à l'opposé (31) comportant chacun une extrémité avant à ancrage automatique moyennant laquelle le dispositif de fixation est capable de sceller une incision traversante dans une paroi oculaire par le biais de laquelle il est implanté dans celle-ci.

6. Dispositif de fixation (10, 20, 30) selon l'une quelconque des revendications 1 à 5, dans lequel ledit élément de support (13) est formé intégralement avec ledit dispositif intraoculaire.

7. Dispositif de fixation (10, 20, 30) selon la revendication 6, dans lequel ledit dispositif intraoculaire administre des médicaments intraoculaires.

8. Dispositif de fixation (10, 20, 30) selon la revendication 6, dans lequel ledit dispositif intraoculaire acquiert des mesures intraoculaires.

9. Dispositif de fixation (10, 20, 30) selon l'une quelconque des revendications 1 à 5, dans lequel ledit élément de support comprend un dispositif de liaison pour supporter un dispositif intraoculaire discret dans l'humeur vitrée d'un oeil.
